# EUROPÄISCHE PATENTSCHRIFT

(11) **EP 1 940 938 B1**
(45) Veröffentlichungstag und Bekanntmachung des Hinweises auf die Patenterteilung: **24.03.2010**
(21) Anmeldenummer: 06792247.6
(22) Anmeldetag: 23.09.2006
(51) Int. Cl.: C08K 5/3432, A61L 29/16

(54) **ANTIMIKROBIELLE KUNSTSTOFFZUSAMMENSETZUNG MIT NIEDRIGER ELUTIONSRATE UND LANGER WIRKSAMKEIT**
SUSTAINED-RELEASE ANTIMICROBIAL PLASTIC COMPOSITION WITH LOW RATE OF ELUTION
COMPOSITION DE MATIERE PLASTIQUE ANTIMICROBIENNE A FAIBLE TAUX D'ELUTION ET A ACTIVITE PROLONGEE

(30) Priorität: 06.10.2005 DE 102005048131
(43) Veröffentlichungstag der Anmeldung: 09.07.2008
(73) Patentinhaber: Bayer Innovation GmbH, 40225 Düsseldorf (DE)
(72) Erfinder: PUDLEINER, Heinz, 47800 Krefeld (DE); HYNER, Joachim, 40764 Langenfeld (DE)
(74) Vertreter: Hildebrand, Steven
(86) Internationale Anmeldenummer: PCT/EP2006/009265
(87) Internationale Veröffentlichungsnummer: WO 2007/039156

(56) Entgegenhaltungen:
- WO-A2-2005/072281
- DE-A1- 1 470 859
- DE-A1- 2 708 331
- DE-A1- 3 908 712
- US-A- 5 328 698
- US-B1- 6 641 831

## Beschreibung

Die vorliegende Erfindung betrifft antimikrobielle Kunststoffzusammensetzungen aus einem thermoplastischen Elastomeren (TPE), besonders thermoplastischen Polyurethanen, und mindestens einem antimikrobiellen Wirkstoff aus der Gruppe der Bis-(4-amino-1-pyridinium)-alkane, speziell Octinidin, die Herstellung dieser Zusammensetzungen sowie die Verwendung dieser Kunststoszusammensetzungen für Katheter und andere medizintechnische Produkte.

Der Einsatz von polymeren organischen Materialien ist aus dem Leben nicht mehr weg zu denken. Naturgegeben sind Werkstücke aus organischen Werkstoffen unter verschiedenen Bedingungen deren Einsatzes empfänglich für die Besiedelung von Mikroorganismen verschiedenster Art wie beispielsweise Bakterien, Viren oder Pilze. Diese führt zu hygienischen und medizinischen Risiken im Umfeld des Werkstückes sowie auch in der Gebrauchstüchtigkeit des Werkstückes selber, letzteres im Falle von unerwünschtem mikrobiologischen Abbau des Materials.

Insbesondere hat der Einsatz von polymeren Materialien für diagnostische und therapeutische Zwecke zu einem signifikanten Technologiesprung in der modernen Medizin geführt. Andererseits hat die häufige Verwendung dieser Materialien in der Medizin zu einem dramatische Anstieg von so genannten Fremdkörperinfektionen respektive Polymer assoziierten Infektionen geführt.

Neben traumatischen und thromboembolischen Komplikationen sind Katheter assoziierte Infektionen bis hin zur Sepsis ein gravierendes Problem beim Einsatz von Venenverweilkathetem (Venous Access Devices) in der Medizin, insbesondere in der Intensivmedizin.

Zahlreiche Studien haben ergeben, dass Koagulase negative Staphylococcen, der transiente Keim Staphylococcus aureus, Staphylococcus epidermis und verschiedene Candida Spezies die Hauptverursacher von Katheter assoziierten Infektionen sind. Diese ubiquitär auf der Haut vorhandenen Mikroorganismen durchdringen bei der Applikation des Katheters die physiologische Hautbarriere und gelangen so in den subkutanen Bereich und letztendlich in die Blutbahn. Die Adhäsion der Bakterien auf der Kunststoffoberfläche wird als essentieller Schritt bei der Pathogenese von Fremdkörperinfektionen betrachtet. Nach der Adhäsion der Hautkeime an der Polymeroberfläche beginnt die metabolisch aktive Proliferation der Bakterien mit der Besiedlung des Polymers. Damit einher geht die Produktion eines Biofilms durch bakterielle Exkretion von extrazellulärer Glykocalix.

Der Biofilm unerstützt die Adhäsion der Erreger und schützt sie vor dem Angriff bestimmter Zellen des Immunsystems. Zudem bildet der Film eine für viele Antibiotika undurchdringliche Barriere. Nach verstärkter Proliferation der pathogenen Keime an der Polymeroberfläche, kann es schließlich zu einer septischen Bakteriämie kommen. Zur Therapie derartiger Infektionen ist eine Entfernung des infizierten Katheters nötig, da eine Chemotherapie mit Antibiotika unphysiologisch hohe Dosen verlangen würde.

Die Häufigkeit von bakteriell induzierten Infektionen bei zentralvenösen Kathetern liegt im Durchschnitt bei ca. 5 %. Insgesamt zeichnen zentralvenöse Katheter für ca. 90 % aller Sepsisfälle in der Intensivmedizin verantwortlich. Die Verwendung von zentralvenösen Kathetern beinhaltet daher nicht nur ein hohes Infektionsrisiko für die Patienten, sondern verursacht auch enorm hohe therapeutische Folgekosten (Nachbehandlung, verlängerte Verweilzeiten in der Klinik, ggf. Invalidität, Tod).

Durch prä-, peri- oder postoperative Maßnahmen (z. B. hygienische Maßnahmen etc.) kann diese Problematik nur teilweise gelöst werden. Eine rationale Strategie zur Prävention von Polymer assoziierten Infektionen besteht in der Modifizierung der verwendeten polymeren Materialien. Ziel dieser Modifizierung muss die Hemmung der Bakterienadhäsion bzw. der Proliferation bereits adhärierter Bakterien sein, um somit kausal Fremdkörperirifektionen zu vermeiden. Dies kann z. B. durch Inkorporierung eines geeigneten Chemotherapeutikums in die Polymermatrix (z. B. Antimikrobiotika wie Antibiotika und Antiseptika) gelingen, vorausgesetzt, dass der eingearbeitete Wirkstoff auch aus der Polymermatrix herausdiffundieren kann. In diesem Fall kann die Freisetzung des antimikrobiellen Wirkstoffes auf einen längeren zeitraum ausgedehnt werden, damit für einen entsprechend längeren Zeitraum die Mikroben- respektive Bakterienadhäsion bzw. deren Proliferation auf dem Polymer verhindert wird.

Methode zur Herstellung antimikrobiell ausgerüsteter Polymere sind bereits bekannt. Hierbei werden die Mikrobizide auf die Oberfläche oder eine Oberflächenschicht auf- oder in den polymeren Werkstoff eingebracht. Für die insbesondere für medizinische Anwendungen verwandten thernioplastischen Polyurethane sind folgende Techniken beschrieben:
a) Adsorption auf der Polymeroberfläche (passiv oder via Surfactants)
b) Einbringen in eine Polymerbeschichtung, die auf der Oberfläche eines Formkörpers appliziert wird
c) Inkorporierung in die bulk-Phase des polymeren Trägermaterials
d) Kovalente Bindung an der Polymeroberfäche
e) Mischen mit einer Polyurethan bildenden Komponente vor der Reaktion zum fertigen Polymer

Aus dem EP 0 550 875 BI geht beispielsweise ein Verfahren hervor, Wirkstoffe in die äußere Schicht medizinischer Artikel einzubringen (Imprägnierung). Dabei wird die implantierbare Vorrichtung aus polymerem Material in einem geeigneten Lösungsmittel gequollen. Die Polymermatrix wird dabei so verändert, dass ein pharmazeutischer Wirkstoff bzw. eine Wirkstoffkombination in das polymere Material des Implantats eindringen kann. Nach Entfernen des Lösungsmittels wird der Wirkstoff in der Polymermatrix eingeschlossen. Nach Kontakt mit dem physiologischen Medium wird der in der implantierbaren Vorrichtung enthaltene Wirkstoff durch Diffusion wieder freigesetzt. Das Freisetzungsprofil kann dabei durch die Wahl des Lösungsmittels und durch Variation der experimentellen Bedingungen in gewissen Grenzen eingestellt werden.

Polymermaterialien für medizinische Anwendungen, die wirkstoffhaltige Beschichtungen aufweisen, werden beispielsweise in dem US P 5,019,096 erwähnt. Beschrieben werden Verfahren zur Herstellung der antimikrobiell wirksamen Beschichtungen sowie Methoden zur Applikation auf die Oberflächen von medizinischen Devices. Die Beschichtungen bestehen aus einer Polymermatrix, insbesondere aus Polyurethanen, Silikonen oder bioabbaubaren Polymeren, und einer antimikrobiell wirksamen Substanz, vorzugsweise einer synergistischen Kombination eines Silbersalzes mit Chlorhexidin oder einem Antibiotikum.

Das US P 5,281,677 beschreibt Blends aus TPU, welche bevorzugt zur Herstellung von mehrlumigen vaskulären Katheter eingesetzt werden. Erwähnt wird, dass die Formkörper auch einen antimikrobiellen Wirkstoff enthalten können, der in einem der Polyurethane vor dem Schmelzprozess gleichgewichtsverteilt (bulk-distnbuted) sein kann.

Das US P 6,120,790 beschreibt thermoplastische Harze, die antimikrobielle oder fungistatische Wirkstoffe enthalten, wobei das Polymer eine Polyetberkette als Baustein enthält. Als Wirkstoffe kämen unter organischen Verbindungen auch Pyridine infrage, ohne dass diese beispielsweise spezifiziert werden.

Die EP 927 222 A1 beschreibt die Einbringung antithrombisch oder antibiotisch wirksamer Substanzen in die Reaktionsmischung zur Herstellung eines TPUs.

Die WO 03/009879 A1 beschreibt Medizinprodukte mit Mikrobiziden in der Polymermatrix, wobei die Oberfläche mit biologischen Oberflächen aktiven Stoffen (engl. biosurfactants) modifiziert ist. Die Wirkstoffe können mit unterschiedlichen Techniken in das Polymer eingebracht werden. Die Oberflächen aktiven Stoffe dienen zur Verringerung der Adhäsion der Bakterien auf der Oberfläche des Formkörpers.

US P 5,906,825 beschreibt Polymere, darunter auch Polyurethane, in denen Biozide respektive Antimikrobiotika (konkret beschrieben sind ausschließlich Pflanzeninhaltsstoffe) in solcher Menge dispergiert sind, dass das Wachstum von Mikroorganismen unterbunden wird, die mit dem Polymer in Berührung kommen. Dieses kann durch Zusatz von einem Agenz optimiert werden, welches die Wanderung und/oder Freisetzung des Biozids reguliert. Erwähnt werden Naturstoffe wie z.B. Vitamin E. Anwendungsschwerpunkt sind Lebensmittelverpackungen.

In Zbl. Bakt. 284, 390-401 (1996) wird die bessere Langzeitwirkung von in eine Silikon- oder Polyurethan-Polymermatrix verteilten Antibiotika gegenüber auf durch Niederschlagstechnik auf die Oberfläche aufgebrachten oder durch Anschwell-Technik in Oberflächennähe eingebrachten Antibiotika beschrieben. Dabei unterliegt die anfänglich hohe Abgaberate des Antibiotikums von der Oberfläche in ein umliegendes wässriges Medium sehr starken, irreproduzierbaren Schwankungen.

Das US P 6,641,831 beschreibt Medizinprodukte mit retardierter pharniakologischer Aktivität,
wobei diese durch Einbringung von zwei Substanzen unterschiedlicher Lipophilie gesteuert wird. Der Kern der Erfindung ist der Effekt dass die Freisetzungsrate eines antimikrobiellen Wirkstoffes durch den Zusatz von einer lipophileren Substanz verringert und die Abgabe somit über einen längeren Zeitraum aufrechterhalten wird. Bevorzugt sei es, dass der Wirkstoff keine hohe Löslichkeit in wäßrigen Medien aufweise. Es wird auch offenbart, dass es möglich ist, die Freisetzung von Desinfektionsmitteln zu verzögern, wobei u. a. Octenidin namentlich genannt wird.

Die JP 08-157641 beschreibt ein Verfahren zur Herstellung von antimikrobiellen Materialien durch Schmelzkneten eines Polymers mit einer spezifischen Oberfläche von größer gleich 17 cm²/g, darunter u. a. Polyurethan, mit einem pulverförmigen Wirkstoffes, bevorzugt Chlorhexidin.

Die CN 1528470 A beschreibt ein Verfahren zur Herstellung eines medizinischen antiinfektiösen Einfühnmgshilfeschlauches für Katheter aus Polyurethan, wobei ein als Muttermaterial bezeichneter Masterbatch, welcher das Antimikrobiotikum enthält, mit dem PU-Rohmaterial gemischt und zum Formkörper extrudiert wird.

In der WO 2004/017738 A werden aus Polymeren und kolloiden, oligodynamischen Agenzien bestehende Zusammensetzungen beschrieben, wobei diese die Bildung eines mikrobiellen Filmes auf der Oberfläche verhindern. Optionell können diese auch weitere pharmazeutische Wirkstoffe enthalten. Unter einer Auflistung einer Vielzahl von beispielsweisen Wirkstoffen sind als typische auch antimikrobielle Wirkstoffe, darunter Octenidin Hydrochlorid erwähnt.

Die antimikrobielle Ausrüstung durch Einsatz von spezifisch wirksamen antibakteriellen Wirkstoffen, den Antibiotika, ist wie deren topische Anwendung in der Medizin wegen der bei systemischer Gabe bekannten Gefahr der Resistenzentwicklung umstritten. Diesem Problem wird in der WO 2005/009495 A abgeholfen, in der die Verwendung von Antiseptika in Polymethylmethacrylat-Knochenzementen offenbart wird. U. a. und nicht bevorzugt werden als mögliche Substanzen Pyridinderivate wie Octenidindihydrochlorid erwähnt, bevorzugt wird Polyhexamethylenbiguanidid (PHMB).

Allen erwähnten Verfahren ist gemeinsam, dass die zeitlich begrenzte Langzeitwirkung der antimikrobiellen Ausrüstung der Formkörper aus polymerem Material, insbesondere von Medizinprodukten beim Einsatz am bzw. im Patienten, optimiert wird. Dieses sowie die Vermeidung der Gefahr einer mikrobiellen Erstinfektion des Formkörpers selber oder von Mensch und Tier durch den Formkörper werden hierbei aber nicht gleichzeitig befriedigend gewährleistet.

Die vorliegende Anmeldung zielt daher besonders auf überwiegend intracorporal angewandt Medizinprodukte ab. Katheter beispielsweise durchdringen die Körperoberfläche für die gesamte Zeit der Anwendung und stellen deshalb ein besonders hohes Risiko für mikrobielle Infektionen dar, wie schon weiter oben dargelegt wurde. Die Gefahr von Erstinfektionen beim Einbringen der Medizinprodukte in den Körper durch mikrobielle Kontamination wird durch die bekannten antimikrobiellen Ausrüstungen noch nicht ausreichend reduziert.

In der DE 27 08 331 C2 (Sterling Drug Inc.) wird die Herstellung von Bis-(4-substituierten-amino-1-pyridinium)-alkanen beschrieben, zu denen auch Octenidin gehört. Als Einsatzgebiet wird die Verhinderung der Bildung von Zahn-Plaque genannt. Polymere werde damit nicht ausgerüstet.

In der EP 1 123 927 A1 wird ein verbessertes Verfahren zur Herstellung der Wirkstoffe aus der Gruppe der Bis-(4-amino-1-pyridinium)-alkane, darunter Octenidin, beschrieben. Als Anwendungsgebiete werden Seifen, Schampoos, Desinfektionsmittel, z. B. zum Desinfizieren der Haut vor Operationen, Anstrichfarben und Lacke genannt. Auf den Einsatz zur Vermeidung von Katheter assoziierten Infektionen wird nicht eingegangen.

Aufgabe der Erfindung war es, antimikrobiell ausgerüstete Kunststoffe, insbesondere diese enthaltende medizinische Artikel wie Katheter, bereitzustellen, die für einen längeren Zeitraum effizient eine Oberflächenbesiedlung durch Keime verhindern und über den Zeitraum von 15 Tagen weniger als 5 % ihrer ursprünglichen Wirkstoffmenge abgeben.

Es wurde nun gefunden, dass sich dies erreichen lässt, wenn Kunststoffzusammcnsetungen aus einem thermoplastischen Elastomer ausgewählt aus der Gruppe bestehend aus Copolyester, Polyether-Block-Amiden und thermoplastischen Polyurethanen eingesetzt werden, die mindestens einen Wirkstoff aus der Gruppe der Bis-[4-(substituierten-amino)-1-pyridinium]-alkane enthalten.

Vorzugsweise sind diese Kunststoffzusammensetzungen so ausgerüstet, dass die Konzentration des Wirkstoffes ausreichend ist, damit über einen längeren Zeitraum die Besiedelung mit unerwünschten Keimen unterbunden, zumindest signifikant reduziert wird. Dieser längere Zeitraum beträgt bevorzugt mindestens 2 Wochen, besonders bevorzugt mehr als 4 Wochen. Unter unerwünschten Keimen werden jeweils bestimmte Bakterien, Viren und Pilze verstanden.

Ein weiterer Gegenstand dieser Erfindung sind Formkörper aus der erfindungsgemäßen Kunststoffzusammensetzung. Solche Formkörper sind beispielsweise Katheter, Schläuche, Folien, Konnektoren, Fasern und Vliese.

Ein weiterer Gegenstand dieser Erfindung ist die Herstellung der erfindungsgemäßen Kunststoffzusammensetzung. Die erfindungsgemäßen Kunststoffzusammensetzungen werden bevorzugt durch thermoplastische Verarbeitung hergestellt und weiterverarbeitet.

Ein weiterer Gegenstand dieser Erfindung ist die Verwendung der erfindungsgemäßen Kunststoffzusammensetzung für Katheter, Schläuche, Folien, Konnektoren, Fasern und Vliese.

Als Wirkstoffe kommen grundsätzlich alle die in der DE 27 08 331 C2. in den Patentansprüchen 1 bis 4 auf S. 28 definierten Wirkstoffe in Frage. Bevorzugt werden die Verbindungen aus den Beispielen 1-82 (S. 5 bis S. 18 Z. 19), besonders bevorzugt wird Octenidin, sein Hydrochlorid oder ganz besonders bevorzugt das Dihydrochlorid 1,1'-(1,10-Decandiyl)bis[4-(octylamino)-pyridinium]-dichlorid eingesetzt.

Diese als Bis-[4-(substituierten-amino)-1-pyridinium]-alkane bezeichneten Wirkstoffe sind durch die allgemeinen Formeln (I) und (II) definiert 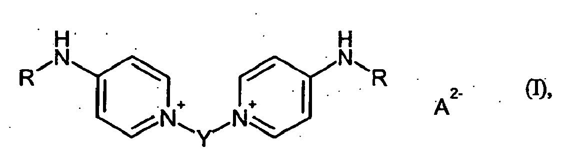 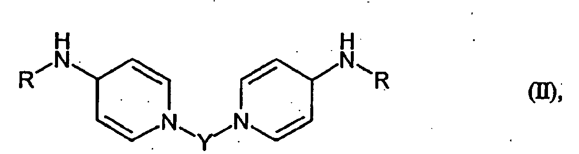 in welchen
- Y: für eine Alkylengruppe mit 4 bis 18 Kohlenstoffatomen steht,
- R: für C₆-C₁₈-Alkyl, C₅-C₇-Cycloalkyl oder durch ein Halogenatom substituiertes Phenyl steht und
- A: für zwei einwertige oder ein zweiwertiges Anion steht.
- Y: steht bevorzugt für 1,10-Derylen oder 1,12-Dodecylen, besonders bevorzugt für 1,12-Dodecylen.
- R: steht bevorzugt für n-Hexyl, n-Heptyl oder n-Octyl, besonders bevorzugt für n-Octyl.
- A: steht beispielsweise für ein Sulfat-, je 2 Fluorid-, Chlorid-, Bromid-, Jodid-, oder Methan- sulfonat-Ionen, bevorzugt für je 2 Fluorid-, Chlorid-, Bromid-, besonders bevorzugt für 2 Chlorid-Ionen.

Die Formel (II) bezeichnet die korrespondierenden freien Basen, welche durch Neutralisation aus den Salzen der Formel (I) nach den üblichen Methoden der organischen Chemie hergestellt werden können. Die Salze der Formel (I) werden in der Literatur häufig auch in Form der Formel (III)

Formel (II) x H₂A (III),

worin "Formel (II)" und A die oben angegebenen Bedeutungen haben, dargestellt. Naturgemäß ist eine chemische Formel nur eine vereinfachte Darstellung der Realität. In diesem Falle sind es Tautomere, für die es keinen Anhaltspunkt gibt, dass diese unter gebräuchlichen Bedingungen und Temperaturen unterscheidbar sind. Für Octenidin-dihydrochlorid gibt es dennoch je 2 Chemical Abstracts Registry Nummern und 2 Nummern im Europäischen Altstoffinventar. Für die Erfindung soll es nicht relevant sein, ob Verbindungen der Formel (I) oder der Formel (III) eingesetzt werden oder in welcher Form diese in der Polymerzusammensetzung vorliegen. Bevorzugt werden Salze der Formel (I) bzw. (III) eingesetzt.

Als Werkstoffe eignen sich thermoplastische Elastomere (TPE) gemäss Anspruch 1. TPE sind Werkstoffe, die elastomere Phasen in thermoplastisch verarbeitbaren Polymeren entweder physikalisch eingemischt oder chemisch eingebunden enthalten. Man unterscheidet Polyblends; denen die elastomeren Phasen physikalisch eingemischt vorliegen und Block-Copolymere in denen die elastomeren Phasen Bestandteil des polymeren Gerüsts sind. Durch den Aufbau der thermoplastischen Elastomere liegen harte und weiche Bereiche nebeneinander vor. Die harten Bereiche bilden dabei eine kristalline Netzstruktur oder eine kontinuierliche Phase deren Zwischenräume von elastomeren Segmenten ausgefüllt sind. Aufgrund dieses Aufbaus haben diese Werkstoffe kautschukähnliche Eigenschaften.

Man kann 3 Hauptgruppen der thermoplastischen Elastomere unterscheiden:
1. Copolyester
2. Polyether-Block-Amide (PEBA)
3. Thermoplastische Polyurethane (TPU)

Verfahren zur Synthese derartiger Copolyester sind aus DE-OS 22 39 271, DE-OS 22 13 128, DE-OS 24 49 343 und US-P 3,023,192 bekannt. Geeignete Copolyester im Sinne der Erfindung sind z. B. auf der Basis von Terephthalsäure mit gewissen Anteilen von Isophthalsäure sowie Butandiol und Polyethern, bevorzugt C₄-Polyethern auf der Basis von Tetrahydrofuran und beispielsweise unter den Handelsnamen Hytrel der Fa. Du Pont, Pelpren der Fa. Toyobo, Arnitel der Fa. Akzo oder Ectel der Fa. Eastman Kodak erhältlich.

Verfahren zur Synthese der PEBA-Polymere sind aus FR-P 7 418 913 (Nr. der Veröffentlichung 2 273 021), DE-OS 28 02 989, DE-OS 28 37 687, DE-OS 25 23 991, EP 0 095 893 B2, DE-OS 27 12 987 beziehungsweise DE OS 27 16 004 bekannt. Erfindungsgemäß bevorzugt geeignet sind solche PEBA-Polymere, die im Gegensatz zu den vorher beschriebenen, statistisch aufgebaut sind. Bausteine sind beispielsweise Adipinsäure, Aminododecansäure, anteilig Hexamethylendiamin, Polytetrahydrofuran, anteilig Polyethylenglykol.

Die erfindungsgemäß einsetzbaren thermoplastisch verarbeitbaren Polyurethane sind durch Umsetzung der polyurethanbildenden Komponenten
A) organisches Diisocyanat,
B) lineares hydroxylterminiertes Polyol mit einem Molekulargewicht von 500 bis 10000,
C) Kettenverlängerer mit einem Molekulargewicht von 60 bis 500,
wobei das Molverhältnis der NCO-Gruppen in A) zu den gegenüber Isocyanat reaktiven Gruppen in B) u. C) 0,9 bis 1,2 beträgt, erhältlich.

Als organische Diisocyanate A) kommen beispielsweise aliphatische, cycloaliphatische, heterocyclische und aromatische Diisocyanate in Betracht, wie sie in Justus Liebigs Annalen der Chemie, 562, S. 75-136 beschrieben werden. Bevorzugt sind aliphatische und cycloaliphatische Diisocyanate.

Im einzelnen seien beispielhaft genannt: aliphatische Diisocyanate, wie Hexamethylendiisocyanat, cycloaliphatische Diisocyanate, wie Isophorondiisocyanat, 1,'4-Cyclohexan-diisocyanat, 1-Methyl-2,4-cyclohexan-diisocyanat und 1-Methyl-2,6-cyclohexan-diisocyanat sowie die entsprechenden Isomerengemische, 4,4'-Dicyclohexylmethan-diisocyanat, 2,4'-Dicyclohexylmethan-diisocyanat und 2,2'-Dicyclohexylmethan-diisocyanat sowie die entsprechenden Isomerengemische, aromatische Diisocyanate, wie 2,4-Toluylendiisocyanat, Gemische aus 2,4-Toluylendiisocyanat und 2,6-Toluylendiisocyanat, 4,4'-Diphenylmethandiisocyanat, 2,4'-Diphenylmethandiisocyanat und 2,2'-Diphenylmethandiisocyanat, Gemische aus 2,4'-Diphenylmethandiisocyanat und 4,4'- Diphenylmethandiisocyanat, Urethan modifizierte flüssige 4,4'-Diphenylmethandiisocyanate und 2,4'-Diphenylmethandiisocyanate, 4,4'-Diisocyanatodiphenyl-ethan-(1,2) und 1,5-Naphthylendiisocyanat. Vorzugsweise verwendet werden 1,6-Hexamethylendiisocyanat, Isophorondiisocyanat, Dicyclohexylmethandiisocyanat, Diphenylmethandiisocyanat-Isomerengemische mit einem 4,4'- Diphenylmethandiisocyanatgehalt von >96 Gew.-% und insbesondere 4,4'-Diphenylmethandiisocyanat und 1,5-Naphthylendiisocyanat. Die genannten Diisocyanate können einzeln oder in Form von Mischungen untereinander zur Anwendung kommen. Sie können auch zusammen mit bis zu 15 Gew.-% (berechnet auf die Gesamtmenge an Diisocyanat) eines Polyisocyanates verwendet werden, beispielsweise Triphenylmethan-4,4',4"-triisocyanat oder Polyphenyl-polymethylen-polyisocyanaten.

Als Komponente B) werden lineare hydroxylterminierte Polyole mit einem mittleren Molekulargewicht Mn von 500 bis 10000, bevorzugt 500 bis 5000, besonders bevorzugt 600 bis 2000 eingesetzt. Produktionsbedingt enthalten diese oft kleine Mengen an verzweigten Verbindungen. Häufig spricht man daher auch von "im wesentlichen linearen Polyolen". Bevorzugt sind Polyether-Diole, Polycarbonat-Diole; sterisch gehinderte Polyesterdiole, hydroxylterminierte Polybutadiene oder Gemische aus diesen.

Als Weichsegmente können allein oder im Gemisch mit den oben genannten Diolen auch Polysiloxan-Diole der Formel (IV)

HO-(CH₂)ₙ-[Si(R¹)₂-O-]ₘSi(R¹)₂-(CH₂)ₙ-OH (IV)

in welcher
- R¹: für eine Alkyl-Gruppe mit 1 bis 6 C-Atomen oder eine Phenyl-Gruppe steht,
- m: für 1 bis 30, bevorzugt 10 bis 25 und besonders bevorzugt 15 bis 25 steht, und
- n: für 3 bis 6 steht,
eingesetzt werden. Es sind bekannte Produkte und können nach an sich bekannten Synthesemethoden hergestellt werden, beispielsweise durch Umsetzung eines Silans der Formel (V)

H-[Si(R¹)₂-O-]ₘSi(R¹)₂-H (V)

in welcher R¹ und m die oben angegebenen Bedeutungen haben,
im Verhältnis 1 : 2 mit einem ungesättigten, aliphatischen oder cycloaliphatischen Alkohol wie Allylalkohol, Buten-(1)-ol oder Penten-(1)-ol in Gegenwart eines Katalysators, z. B. Hexachloroplatinsäure.

Geeignete Polyether-Diole können dadurch hergestellt werden, dass man ein oder mehrere Alkylenoxide mit 2 bis 4 Kohlenstoffatomen im Alkylenrest mit einem Startermolekül, das zwei aktive Wasserstoffatome gebunden enthält, umsetzt. Als Alkylenoxide seien z. B. genannt:

Ethylenoxid, 1,2-Propylenoxid, Epichlorhydrin und 1,2-Butylenoxid und 2,3-Butylenoxid. Vorzugsweise werden Ethylenoxid, Propylenoxid und Mischungen aus 1,2-Propylenoxid und Ethylenoxid eingesetzt. Die Alkylenoxide können einzeln, alternierend nacheinander oder als Mischungen verwendet werden. Als Startermoleküle kommen beispielsweise in Betracht: Wasser, Aminoalkohole, wie N-Alkyl-diethanolamine, beispielsweise N-Methyl-diethanol-amin, und Diole, wie Ethylenglykol, 1,3-Propylenglykol, 1,4-Butandiol und 1,6-Hexandiol. Gegebenenfalls können auch Mischungen von Startermolekülen eingesetzt werden. Geeignete Polyether-Diole sind ferner die hydroxylgruppen-haltigen Polymerisationsprodukte des Tetrahydrofurans. Es können auch trifunktionelle Polyether in Anteilen von 0 bis 30 Gew.-%, bezogen auf die bifunktionellen Polyether, eingesetzt werden, jedoch höchstens in solcher Menge, dass ein thermoplastisch verarbeitbares Produkt entsteht. Die im wesentlichen linearen Polyether-Diole können sowohl einzeln als auch in Form von Mischungen untereinander zur Anwendung kommen.

Geeignete sterisch gehinderte Polyester-Diole können beispielsweise aus Dicarbonsäuren mit 2 bis 12 Kohlenstoffatomen, vorzugsweise 4 bis 6 Kohlenstoffatomen, und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäuren, wie Phthalsäure, Isophthalsäure und Terephthalsäure. Die Dicarbonsäuren können einzeln oder als Gemische, z. B. in Form einer Bernstein-, Glutar- und Adipinsäuremischung, verwendet werden. Zur Herstellung der Polyester-Diole kann es Gegebenenfalls vorteilhaft sein, anstelle der Dicarbonsäuren die entsprechenden Dicarbonsäurederivate, wie

Carbonsäurediester mit 1 bis 4 Kohlenstoffatomen im Alkoholrest, Carbonsäureanhydride oder Carbonsäurechloride zu verwenden. Beispiele für mehrwertige Alkohole sind sterisch gehinderte Glykole mit 2 bis 10, vorzugsweise 2 bis 6 Kohlenstoffatomen, die in beta-Position zur Hydroxylgruppe mindestens einen Alkylrest tragen, wie 2,2-Dimethyl-1,3-propandiol, 2-Methyl-2-propyl-1,3-propandiol, 2,2-Diethyl-1,3-propandiol, 2-Ethyl-1,3-hexandiol, 2,5-Dimethyl-2,5- hexandiol, 2,2,4-Trimethyl-1,3-pentandiol, oder Gemische mit Ethylenglykol, Diethylenglykol, 1,4-Butandiol, 1,5-Pentandiol, 1,6-Hexandiol, 1,10-Decandiol, 1,3-Propandiol und Dipropylenglykol. Je nach den gewünschten Eigenschaften können die mehrwertigen Alkohole allein oder gegebenenfalls in Mischung untereinander verwendet werden. Geeignet sind ferner Ester der Kohlensäure mit den genannten Diolen, insbesondere solchen mit 3 bis 6 Kohlenstoffatomen, wie 2,2-Dimethyl-1,3-propandiol oder 1,6-Hexandiol, Kondensationsprodukte von Hydroxycarbonsäuren, beispielsweise Hydroxycapronsäure und Polymerisationsprodukte von Lactonen, beispielsweise gegebenenfalls substituierten Caprolactonen. Als Polyester-Diole werden vorzugsweise verwendet, Neopentylglykol-polyadipate 1,6-Hexandiol-neopentylglykol-polyadipate. Die Polyester-Diole können einzeln oder in Form von Mischungen untereinander zur Anwendung kommen.

Gegebenenfalls können neben Polyesterdiolen andere Polyole eingesetzt werden zum Beispiel Polycarbonatdiole, Polyetherdiole und Gemische daraus.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diethylenglykol, Triethylenglykol, Tetraethylenglykol oder Thiodiglykol mit Diarylcarbonaten, z. B. Diphenylcarbonat oder Phosgen hergestellt werden können (DE-AS 16 94 080, DE-OS 22 21 751).

Neben den Polyesterpolyolen und den Polycarbonatdiolen können auch Gemische aus Polyetherpolyolen und Polyesterpolyolen und Gemische aus Polyetherpolyolen Polycarbonatdiolen mit jeweils einem zahlenmittleren Molekulargewicht zwischen 600 und 5000 g/mol, bevorzugt 700 und 4200 g/mol eingesetzt werden.

Als Kettenverlängerungsmittel C) werden Diole, Diamine oder Aminoalkohole mit einem Molekulargewicht von 60 bis 500 eingesetzt, vorzugsweise aliphatische Diole mit 2 bis 14 Kohlenstoffatomen, wie z. B. Ethandiol, 1,6-Hexandiol, Diethylenglykol, Dipropylenglykol und insbesondere 1,4-Butandiol. Geeignet sind jedoch auch Diester der Terephthalsäure mit Glykolen mit 2 bis 4 Kohlenstoffatomen, wie z. B. Terephthalsäure-bis-ethylenglykol oder Terephthalsäure-bis-1,4-butandiol, Hydroxyalkylenether des Hydrochinons, wie z. B. 1,4-Di(-hydroxyethyl)-hydrochinon, ethoxylierte Bisphenole, (cyclo)aliphatische. Diamine, wie z. B. Isophorondiamin, Ethylendiamin, 1,2-Propylen-diamin, 1,3-Propylen-diamin, N-Methyl-propylen-1,3-diamin, 1,6-Hexamethylendiamin, 1,4-Diaminocyclohexan, 1,3-Diaminocyclohexan, N,N-Dimethyl-ethylen-diamin und 4,4'-Dicyclohexylmethandiamin und aromatische Diamine, wie z. B. 2,4-Toluylendiamin und 2,6-Toluylen-diamin, 3,5-Diethyl-2,4-toluylen-diamin und 3,5-Diethyl-2,6-toluylendiamin und primäre mono-, di-, tri- oder tetraalkylsubstituierte 4,4'-Diaminodiphenylmethane oder Aminoalkohole wie Ethanolamin, 1-Aminoprapanol, 2-Aminopropanol. Es können auch Gemische der oben genannten Kettenverlängerer eingesetzt werden. Daneben können auch kleinere Mengen an tri- oder höherfunktionellen Vernetzern zugesetzt werden, z. B. Glycerin, Trimethylolpropari, Pentaerythrit, Sorbit. Besonders bevorzugt werden 1,4-Butandiol, 1,6-Hexandiol, Isophorondiamin und deren Gemische eingesetzt.

Weiterhin können in geringen Mengen auch übliche monofunktionelle Verbindungen eingesetzt werden, z. B. als Kettenabbrecher oder Entformungshilfen. Beispielhaft genannt seien Alkohole wie Octanol und Stearylalkohol oder Amine wie Butylamin und Stearylamin.

Die molaren Verhältnisse der Aufbaukomponenten können über einen breiten Bereich variiert werden, wodurch sich die Eigenschaften des Produkts einstellen lassen. Bewährt haben sich molare Verhältnisse von Polyolen zu Kettenverlängerern von 1 : 1 bis 1 : 12. Das Molverhältnis von Diisocyanaten und Polyolen beträgt bevorzugt 1,2 : 1 bis 30 : 1. Besonders bevorzugt sind Verhältnisse von 2 : 1 bis 12 : 1. Zur Herstellung der TPU können die Aufbaukomponenten, gegebenenfalls in Gegenwart von Katalysatoren, Hilfsmitteln und Zusatzstoffe, in solchen Mengen zur Reaktion gebracht werden, dass das Äquivalenzverhältnis von NCO-Gruppen zur Summe der NCO reaktiven Gruppen, insbesondere der Hydroxy- oder Aminogruppen der niedermolekularen Diole/Triole, Amine und der Polyole 0,9 : 1 bis 1,2 : 1, vorzugsweise 0,98 : 1 bis 1,05 : 1, besonders bevorzugt 1,005 : 1 bis 1,01 : 1 beträgt.

Die erfindungsgemäß verwendbaren Polyurethane können ohne Katalysatoren hergestellt werden; in manchen Fällen kann der Einsatz von Katalysatoren jedoch angezeigt sein. Im allgemeinen werden die Katalysatoren in Mengen von bis zu 100 ppm; bezogen auf die Gesamtmenge an Edukten, verwendet. Geeignete erfindungsgemässe Katalysatoren sind die nach dem Stand der Technik bekannten und üblichen tertiären Amine, wie z. B. Triethylamin, Dimethylcyclohexylamin, N-Methylmorpholin, N,N-Dimethyl-piperazin, 2-(Dimethylamino-thoxy)-ethanol, Diazabi-cyclo[2,2,2]octan und ähnliche sowie insbesondere organische Metallverbindungen wie Titansäureester, Eisenverbindungen, Zinnverbindungen, z. B. Zinndiacetat, Zinndioctoat, Zinndilaurat oder die Zinndialkylsalze aliphatischer Carbonsäuren. Bevorzugt sind Dibutylzinndiacetat und Dibutylzinndilaurat; von diesen genügen Mengen von 1 bis 10 ppm, um die Reaktion zu katalysieren.

Neben den TPU-Komponenten und den Katalysatoren können auch andere Hilfsmittel und Zusatzstoffe zugesetzt werden. Genannt seien beispielsweise Gleitmittel wie Fettsäureester, deren Metallseifen, Fettsäureamide und Siliconverbindungen, Antiblockmittel, Inhibitoren, Stabilisatoren gegen Hydrolyse, Licht, Hitze und Verfärbung, Flammschutzmittel, Farbstoffe, Pigmente, anorganische oder organische Füllstoffe und Verstärkungsmittel. Verstärkungsmittel sind insbesondere faserartige Verstärkungsstoffe wie anorganische Fasern, die nach dem Stand der Technik hergestellt werden und auch mit einer Schlichte beaufschlagt sein können. Nähere Angaben über die genannten Hilfs- und Zusatzstoffe sind der Fachliteratur zu entnehmen, beispielsweise J. H. Saunders, K. C. Frisch: "High Polymers", Band XVI, Polyurethane, Teil 1 und 2, Interscience Publishers 1962 bzw. 1964, R. Gächter, H. Müller (Ed.): Taschenbuch der Kunststoff-Additive, 3. Ausgabe, Hanser Verlag, München 1989, oder DE-OS 29 01 774.

Der Aufbau der thermoplastisch verarbeitbaren Polyurethanelastomeren erfolgt bevorzugt schrittweise im so genannten Prepolymerverfahren. Beim Prepolymerverfahren wird aus dem Polyol und dem Diisocyanat ein isocyanathaltiges Prepolymer gebildet, das in einem zweiten Schritt mit dein Kettenverlängerer umgesetzt wird. Die TPU können kontinuierlich oder diskontinuierlich hergestellt werden. Die bekanntesten technischen Herstellverfahren sind das Bandverfahren und das Extruderverfahren.

Die erfindungsgemäßen Formkörper können durch Extrudieren einer Schmelze bestehend aus dem Polymer und Wirkstoff hergestellt werden. Die Schmelze kann 0,01 bis 10 Gew-%, vorzugsweise 0,1 bis 5 Gew.-% Wirkstoff enthalten. Das Vermischen der Komponenten kann nach bekannten Techniken in jeglicher Weise erfolgen. Der Wirkstoff kann beispielsweise direkt in fester Form in die Polymerschmelze eingebracht werden. Es kann auch ein wirkstoffhaltiger Masterbatch direkt mit dem Polymer verschmolzen werden oder mit der bereits vorliegenden Polymerschmelze gemischt werden. Der Wirkstoff kann auch vor dem Schmelzen des Polymers mittels bekannter Techniken auf das Polymer aufgebracht werden (durch Tumbeln, Besprühen etc.). Im übrigen kann die Vermischung/Homogenisierung der Komponenten nach bekannten Techniken über Kneter oder Schneckenmaschinen erfolgen, vorzugsweise in Ein oder Doppelschneckenextrudern in einem Temperaturbereich zwischen 150 und 200°C. Durch das Vermischen der Komponenten während des Extrusionsprozesses, wird eine homogene, niolekulardisperse Verteilung des Wirkstoffs in der Polymermatrix, erzielt, ohne dass zusätzliche Arbeitsschritte erforderlich wären.

Die nachfolgenden Beispiele sollen die Erfindung illustrieren ohne sie jedoch beschränken zu wollen.

### Beispiele

### Beispiel 1 (Vergleichsbeispiel)

Handelsübliches aromatisches Polyetherurethan mit 20 Gew.-% Bariumsulfat: Tecothane TT 2085 A-B20 der Shore-Härte 85 A (Fa. Noveon, Woburn MA)

Das wirkstofffreic Zylindergranulat wurde auf einem Zweiwellenextruder ZSK extrudiert. Es wurde eine klare Schmelze erhalten, die nach Abkühlung im Wasser/Luftbad und Stranggranulation ein farbloses, klares Zylindergranulat ergab.

Für mikrobiologische in vitro Untersuchungen im dynamischen Testmodell sowie für die Bestimmung des Freisetzungsprofils des eingearbeiteten Wirkstoffs wurden Strangproben (2 mm Durchmesser und ca. 17 cm lang) genommen und das Granulat zu Prüfkörpern (Platten) spritzgegossen.

Aus den Platten wurden Plättchen mit 5 mm Durchmesser ausgestanzt. Platten und Strangproben wurde mit 25 kGr Gamma sterilisert.

### Beispiel 2

5 g Öctenidin Dihydrochlorid wurden in einem Intensivmischer auf 995 g wirkstofffreies Tecothane TT2085A-B20 aufgebracht. Das wirkstoffhaltige. Zylindergranulat wurde auf einem Zweiwellenextruder ZSK extrudiert. Es wurde eine klare Schmelze erhalten, die nach Abkühlung im Wasser/Luftbad und Stranggranulation ein farbloses, klares Zylindergranulat ergab:

Für mikrobiologische in vitro Untersuchungen im dynamischen Testmodell sowie für die Bestimmung des Freisetzungsprofils des eingearbeiteten Wirkstoffs wurden Strangproben (2 mm Durchmesser und ca. 17 cm lang) genommen und das Granulat zu Prüfkörpern (Platten) spritzgegossen.

Aus den Platten wurden Plättchen mit 5 mm Durchmesser ausgestanzt. Platten und Strangprobe wurde mit 25 kGr Gamma-sterilisert.

### Beispiel 3

10 g Octenidin Dihydrochlorid wurden in einem Intensivmischer auf 990 g wirkstofffreies Tecothane TT2085A-B20 aufgebracht. Das wirkstoffhaltige Zylindergranulat wurde auf einem Zweiwellenextruder ZSK extrudiert. Es wurde eine klare Schmelze erhalten, die nach Abkühlung im Wasser/Luftbad und Stranggranulation ein farbloses, klares Zylindergranulat ergab.

Für mikrobiologische in vitro Untersuchungen im dynamischen Testmodell sowie für die Bestimmung des Freisetzungsprofils des eingearbeiteten Wirkstoffs wurden Strangproben (2 mm Durchmesser und ca. 17 cm lang) genommen und das Granulat zu Prüfkörpern (Platten) spritzgegossen.

Aus den Platten wurden Plättchen mit 5 mm Durchmesser ausgestanzt. Platten und Strangprobe wurde mit 25 kGr Gamma sterilisert.

### Beispiel 4

15 g Octenidin Dihydrochlorid wurden in einem Intensivmischer auf 985 g wirkstofffreies Tecöthane TT2085A-B20 aufgebracht. Das wirkstoffhaltige Zylindergranulat wurde auf einem Zweiwellenextruder ZSK extrudiert. Es wurde eine klare Schmelze erhalten, die nach Abkühlung im Wasser/Luftbad und Stranggranulation ein farbloses, klares Zylindergranulat ergab.

Für mikrobiologische in vitro Untersuchungen im dynamischen Testmodell sowie für die Bestimmung des Freisetzungsprofils des eingearbeiteten Wirkstoffs wurden Strangproben (2 mm Durchmesser und ca. 17 cm lang) genommen und das Granulat zu Prüfkörpern (Platten) spritzgegossen.

Aus den Platten wurden Plättchen mit 5 mm Durchmesser ausgestanzt. Platten und Strangprobe wurde mit 25 kGr Gamma sterilisert.

### Beispiel 5 (Vergleichsbeispiel)

Kommerziell erhältlicher Katheter, der mit feinteiligem metallischem Silber, Platin und Kohlenstoff antimikrobiell ausgerüstet ist.

### Beispiel 6

Chronoflex AL 85A-B20 wurde bei -40°C zu einem Pulver vermahlen, das anschließend in zwei Fraktionen gesiebt wurde: 1. Fraktion 100 µm bis 300 µm; 2. Fraktion > 300µm

### Beispiel 7

400g Octenidin Dihydrochlorid wurden in einem Intensivmischer mit 3600 g wirkstofffreiem Chronoflex AL 85A-B20-Pulver (100 bis 300 µm) aus Beispiel 6 vermischt. Bei einem Durchsatz des Extruders von 3 kg/Stunde wurden 16 kg Chronoflex AL 85A-B20-Granulat und 4000g der Polymer-Wirkstoff Pulvermischung in Gehäuse 1 des Extruders dosiert. Das wirkstoffhaltige Zylindergranulat wurde auf einem Zweiwellenextruder ZSK der Fa. Brabender extrudiert. Es wurde eine weiße Schmelze erhalten, die nach Abkühlung im Wasser/Luftbad und Stranggranulation ein weißes Zylindergranulat mit 2 Gew.-% Octenidin Dihydrochlorid ergab.

Für die Bestimmung des Freisetzungsprofils des eingearbeiteten Wirkstoffs wurde das Granulat zu Prüfkörpern (Platten) spritzgegossen.

### Beispiel 8

Zur Überprüfung der Wirksamkeit wurde der folgende Versuchsaufbau gewählt:

### Dynamische Modell zum Nachweis antimikrobieller Wirkung von Werkstoffen

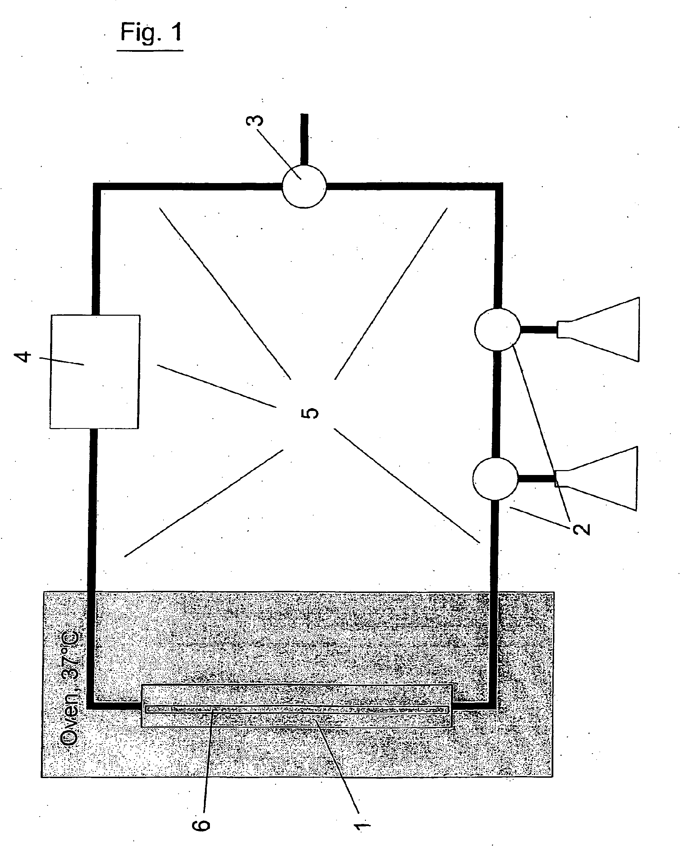

Mit dem vorgestellten Modell soll der Nachweis der antimikrobiellen Wirkung von Werkstoffen geführt und die Verhinderung der Biofilmbildung auf den Werkstoffen nachgewiesen werden. Die Versuchsapparatur besteht aus folgenden Komponenten (vgl. auch Fig 1):

| | |
|---|---|
| 1. | Reaktionskammer |
| 2. | Nährmedien-Austauscher (2 gekoppelte Dreiwegehähne) |
| 3. | Probenkammer |
| 4. | Schlauchpumpe |
| 5. | Schlauchsystem |
| 6. | Probe |

In eine Reaktionskammer wurde ein Strangstück der zu untersuchenden Probe eingebracht und mittels Schrumpfschlauch an beiden Seiten fest fixiert. Die Reaktionskammer ist während der Versuchszeit im Brutschrank positioniert.

Das Schlauchsystem führt weiter zum Nährmedien-Austauscher. Mit einem Dreiwegehahn kann bei Auslaufstellung aus dem Kreislauf Nährmedium abgepumpt, mit dem zweiten kann bei Einlaufstellung in den Kreislauf Nährmedium zugeführt werden.

Das Schlauchsystem führt weiter über die Probenkammer, zur Entnahme von Proben zur Keimzahlbestimmung und Zugabe der Bakteriensuspension, und anschließend über die Schlauchpumpe zurück zur Reaktionskammer.

### 1. Methode

Die Untersuchungen zur Langzeitwirkung der antimikrobiellen Aktivität von Musterproben (Musterschläuche) und Kathetern erfolgte mit Hilfe des Dynamischen-Biofilm-Modells.

### 1.1. Testplatten

Für die Kulturansätze zur Keimzahlbestimmung wurden Mueller-Hinton-Agarplatten verwendet. Dazu wurden Petrischalen von 9 cm Durchmesser mit 18 ml Mueller-Hinton-Agar (Merck KGaA Darmstadt / Lot VM132437 339) gegossen.

### 1.2. Medium

Als Medium für das Dynamischen Biofilm Modell wurde Mueller-Hinton Bouillon ( Merck KGaA Darmstadt / Lot VM205593 347) eingesetzt.

### 1.3. Bakteriensuspension

Die Zugabe des Teststamms in das Dynamische Biofilm Modell erfolgte als Suspension. Aus einer Übernachtkultur vom Teststamm auf Columbia-Blut-Agar wurde eine Suspension mit der Dichte von McFarland 0.5 in NaCl-Lsg. 0.85 % hergestellt. Für die Suspension wurde ein "Kolonie-Pool" aus 3 bis 4 mit der Impföse angetupften Kolonien verwendet. Die Suspension wurde 2x im Verhältnis von 1 : 100 verdünnt. Aus dieser Verdünnungsstufe wurde das Modell befüllt.

### 1.4. Testansatz

Jeder einzelne Modellkreislauf (Reaktionskammer + Schlauchsystem) wurde aus dem mit ihm verbundenem Vorratskolben (Medium 1.2) mit ca. 16 ml Medium befüllt. Danach wurden über die Probenkammer dem Modellkreislauf mit einer Pipette 100 µl der Bakteriensuspension (1.3) zugegeben. Parallel dazu erfolgte das Ausplattieren von 100 µl der Bakteriensuspension zur Keimzahlbestimmung (1.1).

Im Durchschnitt waren nach jeder Zugabe der Bakteriensuspension Keimzahlen von mindestens 200 CPU/ml im Modellkreislauf vorhanden.

Die Schlauchpumpe wurde auf eine Geschwindigkeit von 5 /min (Umläufe pro min.) eingestellt, was bei dem im Versuch verwendeten Schlauch, eine Fördermenge von 0,47 ml/min. ergibt.

Der Inhalt eines Modellkreislaufes wurde damit in der Reaktionskammer in einer guten halben Stunde einmal ausgetauscht bzw. am Katheter vorbeigeführt.

Aus dem Modellkreislauf wurden erstmals nach 24 Stunden, dann täglich oder in variierenden Abständen jeweils 4 ml (25 % der Gesamtflüssigkeit) entnommen und durch neues Medium ersetzt.

In den entnommenen Proben wurde die Bakterienkonzentration in jedem einzelnen Modellkreislauf ermittelt. Aus der Probe wurden mit einer Impföse 50 µl auf eine Testplatte ausgestrichen und 24 Stunden bei 37°C bebrütet. Die Keimzahlen wurden entsprechend dem Wachstum im Ausstrich geschätzt oder es wurden 50 µl mit einer Pipette auf eine Testplatte geimpft, verspatelt, 24 Stunden bei 37°C bebrütet und durch Koloniezählung berechnet.

Zusätzlich zum Medienaustausch wurden täglich oder in variierenden Abständen über die Probenkammer dem Modellkreislauf mit einer Pipette 100 µl der Bakteriensuspension zugegeben. Die Keimzahlen der zugegebenen Bakteriensuspension variierte zwischen 1800 und 15.000 Bakterien pro ml. Auf die Zugabe einer konstanten, immer gleichen Bakterienmenge wurde bewusst verzichtet, da im praktischen Einsatz auch von unterschiedlich vielen Erregern, die Kontakt mit dem Katheter haben könnten, ausgegangen werden muss.

Die zu untersuchenden Strangproben und Katheter wurden am Ende der Versuchszeit nach 30 Tagen aus dem Reaktionsgefäß genommen und in jeweils drei 2 cm lange Stücke geschnitten, die wie folgt aufgearbeitet wurden:
MAKI-Test: Ausrollen jeweils eines Katheterabschnittes auf einer Columbia-Blut Agarplatte 4 mal hin und her.
VORTEX-Test: Der jeweilige Katheterabschnitt wird in 3 ml A.qua dest. 3 mal im Vortex-Schüttler bei 3000/min ( IKA Minishaker ) gewaschen. Aus der Waschlösung werden 3 mal 50 µl mit einer Impföse auf eine Columbia-Blut-Agarplatte ausgestrichen.
ULTRASCHALL-Test:Der jeweilige Katheterabschnitt wird in 3 ml A.. dest. 10 min im Ultraschallbad ultrageschallt und gewaschen. Aus der Waschlösung werden 3 mal 50 µl mit einer Impföse auf eine Columbia-Blut-Agarplatte ausgestrichen.

### 2. Material

### 2.1. Werkstoffproben

Getestet wurden die zur Untersuchung bereitgestellten Strangprobe aus dem Vergleichsbeispiel 1, den erfindungsgemäßen Beispielen 2 bis 4 und dem Vergleichsbeispiel 5.

| | |
|---|---|
| Vergleichbeispiel 1 | Strangprobe |
| Beispiel 2 | Strangprobe |
| Beispiel 3 | Strangprobe |
| Beispiel 4 | Strangprobe |
| Vergleichsbeispiel 5 | Katheterstück |
| | |

### 2.2. Teststämme

Als Teststamm für das Dynamische Biofilm Modell wurde ein für die Biofilmbildung ausgewiesener Staphylococcus epidermidis Stamm ATCC 35984 verwendet. Der Stamm wurde von der Medizinischen Hochschule Hannover zur Verfügung gestellt.

### 3. Auswertung

### 3.1 Biofilmbildung

Es wurde bei 2 Musterschläuchen [Beispiel 1 und Beispiel 6 (beides Vergleichsbeispiele)] eine Bakterienbesiedlung, ein Biofilm beobachtet, bei den übrigen Musterschläuchen konnte kein Bakterienwachstum im Reaktionsmedium, keine Bakterienbesiedlung und kein Biofilm nachgewiesen werden.

### 3.2 Ergebnisdiskussion

Das Dynamische Biofilm Modell ermöglicht den Nachweis der Biofilmbildung oder den Nachweis der Verhinderung der Biofilmbildung durch die antimikrobielle Wirkung eines Werkstoffes bzw. eines fertigen Katheters.

Mit der Versuchsanordnung kann sich der natürliche Situation des Katheters in der Haut angenähert werden.

Folgende Faktoren können annäherungsweise simuliert werden:
- Das Medium enthält alle Faktoren für ein Bakterienwachstum entsprechend der Gewebsflüssigkeit der Haut.
- Der Wirkstoff kann langsam aus dem Katheter in die Umgebung abgegeben werden und dort oder direkt am Katheter antimikrobiell wirksam werden.
- Die Menge der zugeführten Bakterien ist variabel, und kann auf das Niveau der natürlich vorkommenden Mengen oder auf das Niveau einer Infektionsdosis eingestellt werden.

Ausschließlich bei der Strangprobe aus dem Vergleichbeispiel 1 wurden im Medium der Reaktionskammer unterschiedliche hohe Keimzahlen über den gesamten Untersuchungszeitraum von 30 Tagen nachgewiesen werden. Beim Katheter aus Vergleichsbeispiel 5 waren ab dem 7. Versuchstag ständig Bakterien nachweisbar. Bei diesen Proben konnte ebenfalls ein Biofilm nachgewiesen werden.

Bei den Strangproben der erfindungsgemäßen Beispiele 2 bis 4 konnten im Medium der Reaktionskammer bis auf wenige Ausnahmen keine Bakterien über den gesamten Untersuchungszeitraum von 30 Tagen nachgewiesen werden.

Bei den Strangproben der erfindungsgemäßen Beispiele 2 bis 4 werden nach der Zugabe einer hohen Bakterienkonzentration am 28. Versuchstag, am 29. Versuchstag Bakterien in einer Konzentration von 102 pro CFU pro ml im Medium der Reaktionskammer gefunden. Trotzdem sind am nächsten Tag, am 30. Versuchstag, keine Bakterien mehr und auch keine Anhaftung am Musterschlauch und damit auch kein Biofilm nachweisbar.

### Beispiel 9

### Agardiffusionstest

### 1. Methode

Die Untersuchung zur antimikrobiellen Wirkung erfolgte mit Hilfe des Agardiffusionstestes.

### 1.1. Testplatten

Petrischalen von 9 cm Durchmesser wurden mit 18 ml Mueller-Hinton-Agar nach NCCLS (Merck KGaA Darmstadt / Lot ZC217935 430) gegossen.

### 1.2. Bakteriensuspension

Aus einer Übemachtkultur vom Teststamm auf Columbia-Blut-Agar wurde eine Suspension mit der Dichte von McFarland 0.5 in NaCl-Lsg. 0.85% hergestellt..Für die Suspension wurde ein "Kolonie-Pool" aus 3 bis 4 mit der Impföse angetupften Kolonien verwendet.

### 1.3. Testansatz

In die Suspension wird ein steriler Wattetupfer getaucht. Der Flüssigkeits-Überschuss wird am Glasrand ausgedrückt. Mit dem Tupfer wird die Mueller-Hinton Agarplatte in drei Richtungen im Winkel von jeweils 60° gleichmäßig beimpft. Danach werden Werkstoffplättchen und Testplättchen auf die Testplatte aufgelegt. Die Testplatten wurden bei 37° C für 24 Stunden bebrütet.

Die antimikrobielle Wirkung der Proben wurde an Hand von Hemmhöfen beurteilt.

Ein Vergleich mit den Untersuchungen im Agardiffusionstest, indem alle Proben auf ihre antimikrobielle Wirkung getestet wurden, zeigt, dass die Proben, die bei dieser Untersuchung mit kaum einer, bzw. gar keiner antimikrobiellen Wirkung aufgefallen sind, ebenfalls keine antimikrobielle Wirkung zeigen und mit einem starken Biofilm behaftet sind (vgl. Tabelle 1).

**Tabelle 1: Mikrobiologische Wirksamkeit im Agardifusionstest gegen verschiedene Keime**

| | | | | | | | |
|---|---|---|---|---|---|---|---|
| Teststamm | E. coli | P. mirabilis | P. aeruginosa | S. aureus | MRSA | C. albicans | |
| Material | 35218 | 35695 | 27853 | 29213 | 0134-93 | 14053 | Biofilm |
| Vergleichsbeispiel 1 | - | - | - | - | - | - | + |
| Beispiel 2 | + | + | + | + | + | + | - |
| Beispiel 3 | + | + | + | + | + | + | - |
| Beispiel 4 | + | + | + | + | + | + | - |
| Vergleichsbeispiel 5 | - | - | - | - | - | - | + |

| | | | | | | | |
|---|---|---|---|---|---|---|---|
| - keine Wirksamkeit (letzte Spalte: keine Biofilmbildung) + Wirksamkeit (letzte Spalte: Biofilmbildung) | | | | | | | |

Die Proben aus den erfindungsgemäßen Beispielen 2 bis 4 haben darüber hinaus noch die Fähigkeit, nicht nur die Besiedlung durch gramnegativen und grampositiven Bakterien, sondern auch durch Spiosspilze zu verhindern.

### Beispiel 10

Der Elutionsversuche wurden mit Spritzgußplatten, die in 1 cm² große Stücke zerschnnitten wurden, durchgeführt. Die Proben wogen jeweils ca. 2,2 g und hatten eine Oberfläche von 20,5 cm².

Als Elutionsmedium wurden 16 ml demineralisiertes Wasser verwendet. Nach 1 h, 4 h, 8 h, 24 h, 48 h, 120 h und 360 Stunden (15 Tage) wurde das Elutionsmedium Wasser jeweils gegen neues ausgetauscht und der Wirkstoff-Gehalt in den Lösungen bestimmt.

**Tabelle 2: Eluierte Wirkstoff-Menge bezogen auf die ursprüngliche vorhandene Menge**

| **Stunden** | Beispiel 2 | Beispiel 3 | Beispiel 4 | Beispiel 7 |
|---|---|---|---|---|
| **1** | 0,089% | 0,227% | 0,100% | 0,023% |
| **4** | 0,207% | 0,459% | 0,310% | 0,025% |
| **12** | 0,326% | 0,615% | 0,506% | 0,027% |
| **24** | 0,622% | 1,067% | 0,972% | 0,029% |
| **48** | 1,096% | 1,600% | 1,497% | 0,031% |
| **120** | 2,296% | 3,059% | 2,980% | 0,039% |
| **360,** | 5,200% | 6,711% | 6,340% | 0,108% |

Summiert über alle 7 Lösungen waren nach 15 Tagen aus dem Plättchen des Beispiels 2 5,200 %, aus den Plättchen des Beispiels 3 6,711 %, aus den Plättchen des Beispiels 4 6,34 % und aus den Plättchen des Beispiels 7 sogar erst 0,108% der ursprünglichen Wirkstoffmenge extrahiert worden.

### Beschreibung der Abbildung

Fig. 1 Bauteile der Versuchsapparatur für das dynamische Modell Beispiel 8 mit Probe:

| | |
|---|---|
| 1 | Reaktionskammer |
| 2 | Nährmedien-Austauscher (2 gekoppelte Dreiwegehähne) |
| 3 | Probenkammer |
| 4 | Schlauchpumpe |
| 5 | Schlauchsystem |
| 6 | Probe |

## Patentansprüche

1. Kunststoffzusammensetzung enthaltend ein thermoplastisches Elastomer ausgewählt ist aus der Gruppe bestehend aus Copolyester, Polyether-Block-Amiden und thermoplastischen Polyurethanen und mindestens einen Wirkstoff aus der Gruppe der Bis-(4-substituierten-amino-1-pyridinium)-alkane.

2. Kunststoffzusammensetzung gemäß Anspruch 1, **dadurch gekennzeichnet, dass** der Wirkstoff ausgewählt ist aus der Gruppe bestehend aus Stoffen der allgemeinen Formeln (I) und (II) 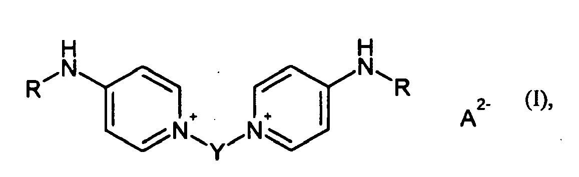 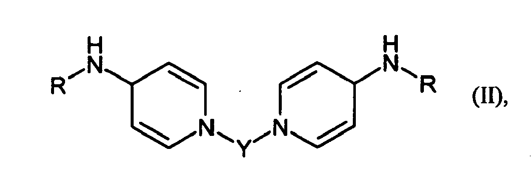 in welchen
Y für eine Alkylengruppe mit 4 bis 18 Kohlenstoffatomen steht,
R für C₆-C₁₈-Alkyl, C₅-C₇-Cycloalkyl oder durch ein Halogenatom substituiertes Phenyl steht und
A für zwei einwertige oder ein zweiwertiges Anion steht.

3. Kunststoffzusammensetzung gemäß einem der vorstehenden Ansprüche, **dadurch gekennzeichnet, dass** die Konzentration des Wirkstoffes ausreichend ist, damit über einen längeren Zeitraum die Besiedelung mit unerwünschten Keimen unterbunden oder signifikant reduziert wird.

4. Kunststoffzusammensetzung gemäß einem der vorstehenden Ansprüche, **dadurch gekennzeichnet, dass** die Konzentration des Wirkstoffes 0,01 bis 5 Gewichtsprozent bezogen auf Wirkstoffes und thermoplastisches Elastomer beträgt.

5. Kunststoffzusammensetzung gemäß einem der vorstehenden Ansprüche, **dadurch gekennzeichnet, dass** er aus thermoplastischem Polyurethan und Octenidin Dihydrochlorid besteht.

6. Verfahren zur Herstellung einer Kunststoffzusammensetzung gemäß einem der Ansprüche 1 bis 5 umfassend das Extrudieren einer Schmelze aus Wirkstoff und thermoplastischem Elastomer.

7. Formkörper enthaltend eine Kunststoffzusammensetzung gemäß einem der Ansprüche 1 bis 5.

## Claims

1. Plastics composition comprising a thermoplastic elastomer selected from the group consisting of copolyester, polyether block amides and thermoplastic polyurethanes and comprising at least one active ingredient from the group of the bis(4-(substituted amino)-1-pyridinium)alkanes.

2. Plastics composition according to Claim 1, **characterized in that** the active ingredient has been selected from the group consisting of substances of the general formulae (I) and (II) 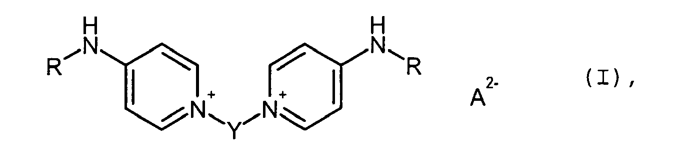 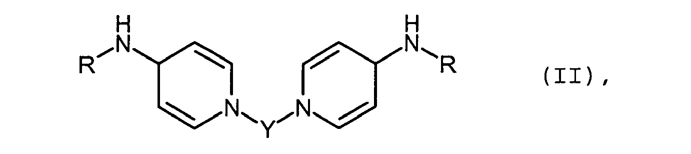 where
Y is an alkylene group having from 4 to 18 carbon atoms,
R is C₆-C₁₈-alkyl, C₅-C₇-cycloalkyl or halogen-atom-substituted phenyl and
A is two monovalent anions or a divalent anion.

3. Plastics composition according to any of the preceding claims, **characterized in that** the concentration of the active ingredient is sufficient to suppress or significantly reduce, over a prolonged period, colonization by undesired microbes.

4. Plastics composition according to any of the preceding claims, **characterized in that** the concentration of the active ingredient is from 0.01 to 5 per cent by weight, based on active ingredient and thermoplastic elastomer.

5. Plastics composition according to any of the preceding claims, **characterized in that** it is composed of thermoplastic polyurethane and octenidine dihydrochloride.

6. Process for preparation of a plastics composition according to any of Claims 1 to 5 encompassing extrusion of a melt composed of active ingredient and of thermoplastic elastomer.

7. Moulding comprising a plastics composition according to any of Claims 1 to 5.

## Revendications

1. Composition de matière plastique contenant un élastomère thermoplastique choisi dans le groupe composé de copolyesters, de polyéthers-blocs-amides et de polyuréthanes thermoplastiques et au moins un principe actif du groupe des bis-(amino-1-pyridinium substitué en 4)-alcanes.

2. Composition de matière plastique selon la revendication 1, **caractérisée en ce que** le principe actif est choisi dans le groupe composé de substances des formules générales (I) et (II) 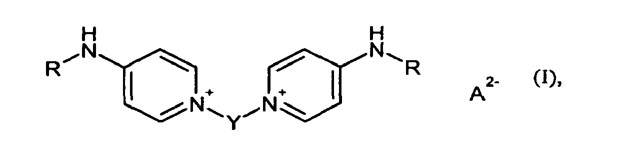 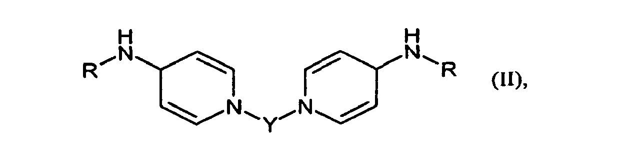 où
Y représente un groupe alkylène ayant de 4 à 18 atomes de carbone,
R représente un alkyle en C₅-C₁₈, un cycloalkyle en C₅-C₇ ou un phényle substitué par un atome d'halogène et
A représente deux anions monovalents ou un anion divalent.

3. Composition de matière plastique selon l'une des revendications précédentes, **caractérisée en ce que** la concentration du principe actif est suffisante pour inhiber ou réduire de façon significative la colonisation par des gènes non désirés pendant une période prolongée.

4. Composition de matière plastique selon l'une des revendications précédentes, **caractérisée en ce que** la concentration du principe actif est de 0,01 à 5 % en poids par rapport au principe actif et à l'élastomère thermoplastique.

5. Composition de matière plastique selon l'une des revendications précédentes, **caractérisée en ce qu'**elle est composée de polyuréthane thermoplastique et de dichlorhydrate d'octénidine.

6. Procédé de préparation d'une composition de matière plastique selon l'une des revendications 1 à 5 comprenant l'extrusion d'une masse fondue composée du principe actif et de l'élastomère thermoplastique.

7. Corps moulé contenant une composition de matière plastique selon l'une des revendications 1 à 5.
